# EUROPEAN PATENT SPECIFICATION

(11) **EP 0 474 757 B1**
(45) Date of publication and mention of the grant of the patent: **21.09.1994**
(21) Application number: 90909206.6
(22) Date of filing: 10.05.1990
(51) Int. Cl.: A61F 13/22

(54) **A TAMPON AND A METHOD FOR ITS MANUFACTURE**
TAMPON SOWIE VERFAHREN ZU SEINER HERSTELLUNG
TAMPON ET PROCEDE POUR SA FABRICATION

(30) Priority: 30.05.1989 SE 8901936
(43) Date of publication of application: 18.03.1992
(73) Proprietor: SanPoint Innovation AB, S-647 00 Mariefred (SE)
(72) Inventor: HODEN, Ebbe, S-647 00 Mariefred (SE); MOKVIST, Maja, Snellville, GA 30278 (US)
(74) Representative: Nilsson, Karl Ingvar
(86) International application number: PCT/SE90/00313
(87) International publication number: WO 90/14811

(56) References cited:
- SE-B- 356 901
- SE-B- 413 979
- SE-B- 419 598

## Description

The present invention relates to an improved tampon, particularly a menstruation tampon, of the kind set forth in the preamble of claim 1 (see also SE-B-413979). The invention also relates to a method for the manufacture of the improved tampon according to claim 1.

An object of the present invention is to provide a novel and advantageous tampon whose construction enables the absorbent filling of the tampon to be selected from a wide choice of materials, for example so that a highly absorbent filling material which has little or substantially negligible self-cohesion can be used in the tampon in a problem-free manner.

To this end, there is proposed in accordance with the invention a tampon of the kind in question and exhibiting the features specified in the characterizing clause of claim 1.

As before mentioned, the invention also provide-s a novel and advantageous tampon-manufacturing method, this method comprising forming a tampon blank in the form of a body having a substantially circular cross-sectional shape and containing a filling of absorbent material enclosed in a casing of thin, liquid-permeable layer material, and compacting the tampon blank to its final tampon form, and in which method the absorbent material forming said filling is applied in the form of a string which has a width which corresponds at least substantially to the length of the finished tampon onto a strip of said layer material which has a greatest width exceeding the width of the string, said string being placed on the strip in spaced relationship with the longitudinal edges of said strip, wherein
the strip of layer material having located thereon the string of absorbent material, for forming said tampon blank, is coiled several turns onto a first tubular mandrel with said string located on the side of the strip facing towards said mandrel;
a part of the strip projecting from one side of the string with the end of the mandrel located adjacent the inner edge of said part is inserted into the interior of said first mandrel;
a second tubular mandrel is inserted through said mandrel end of the first mandrel in a manner such as to be surrounded by the layer-material part inserted into said first mandrel;
the first mandrel is then completely withdrawn;
a part of the strip projecting out from the string on the other side, with the end of the second mandrel located adjacent the inner end of said part, is inserted into the interior of the second mandrel; and the second mandrel is then fully withdrawn prior to compacting the tampon blank thus formed to its final tampon form.

Further characterizing features of the invention are set forth in the depending Claims 2 to 9 and 11 to 18, whereas further advantages afforded by the invention will be apparent from the following description made with reference to the accompanying drawings, in which
Figure 1 illustrates highly schematically a first exemplifying embodiment of a machine for manufacturing tampons in accordance with the invention;
Figures 2a-d illustrate highly schematically the various stages in the manufacture of a first embodiment of the tampon according to the invention;
Figure 3 illustrates highly schematically part of a second embodiment of a machine for the manufacture of a second embodiment of the tampon in accordance with the invention;
Figure 4 is a view from above of part of the web of layer material located between the web-cutting station and the conical winding mandrel shown in Figure 3, with an arcuately curved string of material positioned on the web; and
Figure 5 is a view which corresponds to the view of figure 2a and which illustrates an introductary phase in the second embodiment of manufacture of the inventive tampon.

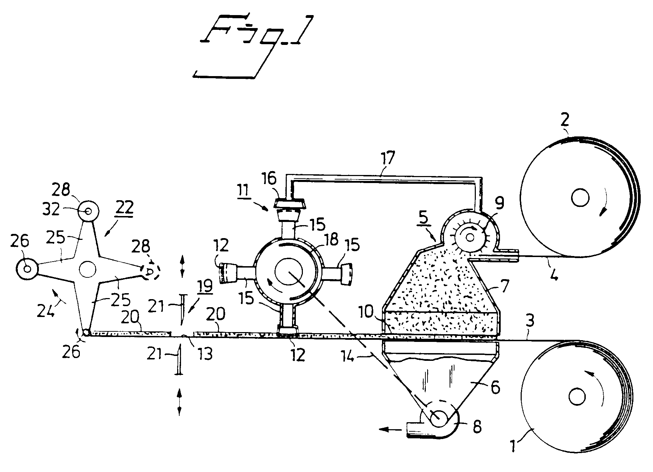

The reference numerals 1 and 2 in Figure 1 identify storage reels comprising respectively thin strip-like liquid-permeable layer material and strip-like starting material for an absorbent-filling enclosed in the layer material in tampons produced by the illustrated machine. Webs 3 and 4 of the layer material and the tampon-filling starting material are drawn from respective reels 1, 2, preferably continuously, with the reels rotating in the direction shown by the arrows. The web 3 of layer material may advantageously consist of a nonwoven material, such as nonwoven gauze, and the web 4 forming the starting material for the tampon filling is here presumed to consist of a suitable paper pulp, so-called fluff pulp. The web 3 is conveyed through the machine while resting on an endless wire belt (not shown) or some like device and is passed to a first station 5 comprising lower hood 6 and an upper hood 7. The hood 6 is connected at the bottom thereof to the suction side of a fan 8, and a shredder or defibrator 9 is connected to the top of the hood 7. When seen at right angles to the plane of the drawing, the hoods 6 and 7 have a width which is smaller than the width of the web 3, preperably less than half the width of said web 3. The fan 8 is operative to generate a subpressure in the hood 6, so that the discrete pulp-fibres produced in the defibrator 9 will be sucked firmly against the web 3, to form a fibre string 10 of high absorbency, which accompanies the web 3 out of the station 5.

The reference 11 identifies generally a suction station in which parts 12 of the fibre-string 10 are removed from the string by suction at uniform intervals, such as to leave parts 13 on the web 3 which are devoid of fibre material formed from the reel 2. The suction station has the form of a hollow wheel which rotates in the direction of the arrow shown and the interior of which is connected to a subpressure source, for instance to the suction side of the fan 8, as indicated by the broken line 14. The wheel is provided with radially extending, hollow arms 15, the outer ends of which are perforated and move at a peripheral speed which coincides with the speed at which the web 3 and the string 10 move from the right to the left in Figure 1. The string-parts 12 entrained by the arms 15 are sucked through a hood 16 as the arms pass said hood. The hood 16 is connected to the hood 7 in the station 5 via a conduit 17 and the defibrator 9. In the illustrated embodiment, there is provided a stationary shielding plate 18 which will ensure that a subpressure will prevail at the outer, perforated ends of the arms 15 solely while the arms move from the lowermost position of their rotating cycle to a position immediately before the hood 16.

The web 3 is cut at the location of the empty web-parts 13 in a web-cutting station 19 located downstream of the station 11, such as to separate the web 3 between the residual string-parts 20 located thereon. Cutting of the web is effected by means of two mutually coacting cutters 21 which move reciprocatingly in the direction of the arrows shown.

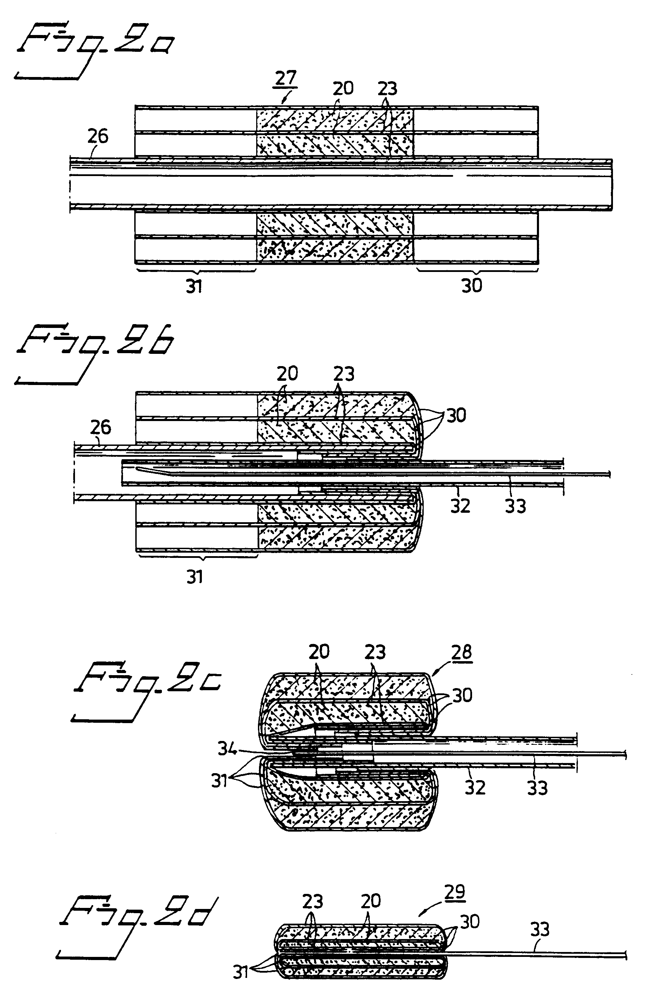

The machine includes a terminal station 22, in which the web-pieces or strips 23 (Figures 2a-d) separated in the cutting station 19 together with the loose-fibre string-parts 20 carried thereon are wound onto rotatable first hollow mandrels 26 which are displaceable in the direction of their longitudinal axis, i.e. in a direction perpendicular to the plane of the drawing in Figure 1. These mandrels 26 are attached to the outer ends of arms 25 which extend radially outwards from a hub part of a wheel which rotates intermittently in the direction of arrow 24. In the lowermost position in the station 22, the mandrels 26 rotate clockwise in the manner indicated by an arrow, and the peripheral speed of the mandrel 26 or the strip 23 carrying the string-part 20 rolled thereon exceeds the speed of the web 3, so as to create a distance between the rearward end of the strip being wound and the forward end of the next-following strip. This arrangement enables the wheel carrying said arms 25 to rotate one-quarter of a revolution at a time in the direction of the arrow 24, thereby enabling a fresh mandrel 26 to be brought to the lowermost position in readiness for receiving and winding each arriving strip 23 carrying a string-part 20.

Thus, in the first or lowermost mandrel position in the station 22, a strip 23 carrying a string-part 20 is wound onto a hollow, rotable mandrel to form a roll or cylinder 27 having the configuration illustrated in Figure 2a. Subsequent to rotating through 90° to a second position and through 180° to a third position, the first and the second ends of the roll 27 are treated in the manner illustrated in Figures 2b and 2c. Subsequent to rotating through 270° to a fourth position, the finished tampon blank is removed from the station 22, this tampon blank being shown in broken lines at 28 in Figure 1 and in Figure 2c. The tampon blank is then compressed, predominently in a radial direction, in a conventional manner (for example in accordance with SE Patent Specification 8800774-5, Figure 2, corresponding to WO 89/07924) to form a finished tampon 29, illustrated in Figure 2d.

The roll or cylinder 27 illustrated in Figure 2a comprises two turns or windings of the string 20 of absorbent filling material, these turns being separated or covered respectively by three turns of the strip 23 of liquid-permeable layer material. The string 20 of filling material has a width which corresponds essentially to the length of the finished tampon shown in Figure 2d. The tubular mandrel 26 extends from the left in Figure 2a, completely through the roll 27, including the parts 30 and 31 of the strip 23 projecting from both sides of the string 20.

Figure 2b corresponds to the aforesaid second mandrel position in station 22 in Figure 1. In this position, the mandrel 26 has been withdrawn so that its forward end terminates at the inner edge of the strip-part 30, and this strip-part 30 has been inserted into the interior of the mandrel 26 together with or by means of a second tubular mandrel 32 whose outer diameter is smaller than the inner diameter of the mandrel 26 such as to provide space between the mandrels 26, 32 for accommodation of the inserted strip-part 30 and such that said strip-part will surround the second mandrel 32. The second mandrel 32 is inserted into the first mandrel 26 from the right in Figure 2b, and a withdrawal thread 33 is inserted into the second mandrel 32, the end of the second mandrel and the end of the thread 33 being located in the proximity of the outer edge of the strip-part 31.

Figure 2c corresponds to the said third position in the station 22 in Figure 1, in which the mandrel 26 has been fully withdrawn and the second mandrel 32 has been drawn back to a position in which its forward end is located adjacent the inner edge of the strip-part 31. Furthermore, the outer end of the withdrawal thread 33 has been connected at 34 to the strip-part 31, and the strip-part 31 has been inserted, e.g. by means of a third mandrel (not shown), or by means of the withdrawal thread 33 into the second mandrel 32. The tampon blank 28 formed in accordance with Figure 2c is removed from the second mandrel 32 (in the fourth position in the station 22 in the Figure 1) and is compressed or compacted in a conventional manner, to form the finished tampon 29 illustrated in Figure 2d. It will be seen that the illustrated construction of the tampon 29 and the illustrated method of attachment of the withdrawal thread 33 prevents effectively axial movement of the winding turns relative to one another, and that a filling 20 of highly-absorbent, discrete particles or fibres, which otherwise have the tendency to lump together locally, is effectively reinforced and prevented from forming agglomerates by the illustrated arrangement of the strip 23, which may itself consist of a material of low absorbency or a totally inabsorbent material. As a result of the illustrated arrangement, there is obtained circumferential passages of substantially uniform thickness which are filled by the filling material 20, which is a highly advantageous pattern from the aspect of absorption.

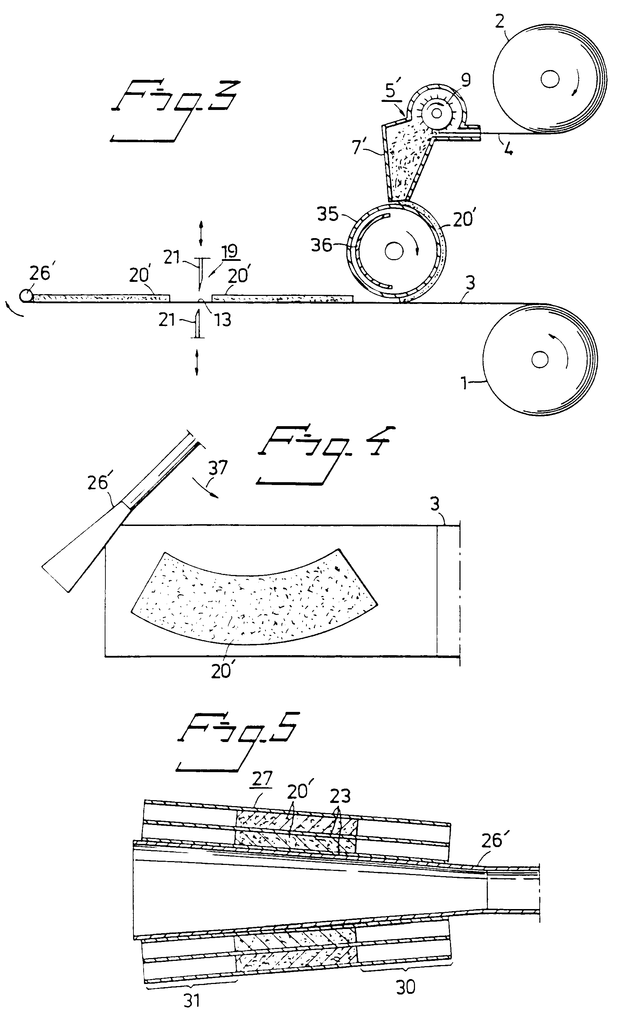

In the case of the modified tampon manufacturing machine shown partially in figure 3, webs of, e.g., non-woven and fluff pulp 3 and 4 are drawn from a respective reel 1 and 2. The web 3 is assumed to rest on an endless cloth or the like and is introduced into a first station 5', in which material strings 20' are deposited in mutually spaced relationship onto the web 3, which is preferably advanced continously. Mounted in the station 5' is a hollow wheel 35 whose interior is connected to a source of subpressure (not shown). The cylindrical surface of the wheel 35 is perforated in regions corresponding to the area and configuration of the strings 20'. Arranged immediately above the wheel 35 is a hood 7' which collects the absorbent fibres that have been formed by defibration of the web 4 drawn from the reel 2, in a shredder or defibrator 9. When the wheel 35 rotates in a clockwise direction as seen in Figure 3, the subpressure prevailing in the wheel causes the fibres to be deposited on the cylindrical surface of the wheel in the form of a material string 20' whose configuration corresponds to the configuration of the pattern of the perforations in said surface. A stationary screening plate 36 mounted in the wheel 35, prevents the subpressure in the wheel from acting on that part of the cylindrical surface covered by said plate. As a result, strings 20' formed on the cylindrical surface of said wheel are deposited on the web 3 and accompany the web in its further movement. Those parts 13 of the web located between sequential strings 20' are severed in the cutting station 19 by means of the cutters 21.

The blanks in the form of web pieces or strips 23 with string parts 20' located thereon obtained when severing the web are wound and shaped into tampons in essentially the same manner as that described with reference to Figure 1 and Figures 2a-d, and hence the method of tampon manufacture with respect to the modified machine will only be described with regard to those aspects which deviate from the method first described.

As shown in Figure 4, the strings 20' can be given a shape other than rectangular. The thicknesses of the strings can also be varied as desired, for instance by varying the number and/or size of the perforations in the cylindrical outer surface of the wheel 35. Because the strings of the Figure 4 embodiment have an arcuately curved configuration, there is used a conical first winding mandrel 26'. As the tampon blank is wound onto the mandrel 26', the mandrel and the tampon blank carried thereby are caused to move arcuately in relation to one another, as indicated by the arrow 37, such that the mandrel axis will constantly form essentially a right angle with the longitudinally extending arcuate edges of the strings 20', and such that the string turns wound on the mandrel will be located essentially centrally above one another, as illustrated in Figure 5. Manufacture of tampons is continued, by drawing-in the edge-parts 30 and 31 (Figure 5) of the strip 23 and attaching a withdrawal thread in the manner described above with reference to Figures 2b-d.

The method described with reference to Figures 4 and 5 provides a substantially cylindrical tampon which nevertheless has a tendency to return to its original essentially conical shape. This is particulary beneficial in the case of menstruation tampons, since it affords improved security against leakage between the tampon on the walls of the vagina. This benefit is particularly accentuated when the string 20', or at least the longer arcuate side of the string, includes material which exhibits an intrinsic elasticity that is substantially independent of moisture conditions, such that the tampon will have a pronounced tendency to return to the shape that it had prior to being compressed, even when substantially dry, i.e. before being moistered by menstruation fluid. In this regard, the string 20' is advantageously thinner towards its longer arcuate side, for instance so that the amount of material in the tampon is not changed along the length of the tampon to any appreciable extent.

It will be understood that the invention is not restricted to the illustrated and described embodiments, but that modifications can be made within the scope of the inventive concept defined in the following Claims. For example, the filling 20 may comprise more than one material and may consist initially of loose absorbent particles and/or fibres. Furthermore, the layer material may be composed of more than one web and the web or webs may have widths which vary along their respective lengths, such that the width of the forward ends of the strips 23 corresponds to the width of the string 20, whereas the width of the rear ends of the strips 23 have a width considerably greater than the width of the string 20.

## Claims

1. A tampon, comprising a body of substantially circular cross-sectional shape formed by a string (20;20') of absorbent material having a width which corresponds substantially to the length of the tampon and wound several turns around an axis extending transversely to the longitudinal direction of said string, and by thin, liquid-permeable layer material (23) surrounding the wound string (20;20') and having an extension in the direction of said axis greater than the length of the tampon, wherein portions (30,31) of the layer material which extend externally to both sides of the wound string are twisted inwardly and accommodated in a space located inwardly of the innermost turn to form a roll which is then compressed predominantly in its radial direction to form said body, characterized in that said layer material (23) comprises an elongate strip which has been wound together with said string (20;20') in a manner to form a roll, the turns of which comprise single layers of said strip (23) with a filling formed from the string (20;20') of absorbent material located between said layers.

2. A tampon according to claim 1, wherein the string (20') is arcuately curved and wherein the tampon blank is wound to a truncated conical roll-like shape in which the string turns separated by a single layer or strip (23) are located at least substantially centrally above one another.

3. A tampon according to claim 2, wherein the string (20') is thinned at the longer arcuate side thereof.

4. A tampon according to claim 2 or claim 3, wherein the string (20') contains, at least adjacent the longer arcuate side thereof, a material which exhibits an intrinsic elasticity which is essentially independent of moist conditions such that the tampon will tend to expand and return to the shape that it had prior to being compressed, even when in a substantially dry state.

5. A tampon according to any one of Claims 1-4, wherein said string (20;20') is comprised at least partially of discrete particles or fibres of absorbent material.

6. A tampon according to Claim 5, wherein at least an essential part of said absorbent material consists of defibrated paper pulp or fluff pulp.

7. A tampon according to Claim 6, wherein said pulp is unbleached.

8. A tampon according to any one of Claims 1-7, wherein said string (20;20') terminates at a distance from at least one end of the layer-material strip (23).

9. A tampon according to any one of Claims 1-8, wherein one end part of a withdrawal thread (33) extends in through one end of the tampon (29) surrounded by the inwardly twisted strip-part (30) at said end and is fastened to the strip-part (31) twisted inwardly at the opposite end of the tampon and located in the tampon inwardly of the innermost turn.

10. A method for manufacturing a tampon according to claim 1, comprising formig a tampon blank (28) in the form of a body having a substantially circular cross-sectional shape and containing a filling of absorbent material enclosed in a casing of thin, liquid-permeable layer material, and compacting the tampon blank to its final tampon form, and in which method the absorbent material forming said filling is applied in the form of a string (20;20') which has a width which corresponds at least substantially to the length of the finished tampon (29) onto a strip (23) of said layer material which has a greatest width exceeding the width of the string (20;20'), said string being placed on the strip in spaced relationship with the longitudinal edges of said strip, wherein:
the strip (23) of layer material having located thereon the string (20;20') of absorbent material, for forming said tampon blank (28), is coiled several turns onto a first tubular mandrel (26:26') with said string located on the side of the strip facing towards said mandrel;
a part (30) of the strip (23) projecting from one side of the string (20;20') with the end of the mandrel located adjacent the inner edge of said part is inserted into the interior of said first mandrel (26;26'); a second tubular mandrel (32) is inserted through said mandrel end of the first mandrel (26;26') in a manner such as to be surrounded by the layer-material part (30) inserted into said first mandrel;
the first mandrel is then completely withdrawn;
a part (31) of the strip (23) projecting out from the string (20;20') on the other side, with the end of the second mandrel (32) located adjacent the inner edge of said part, is inserted into the interior of the second mandrel; and
the second mandrel (32) is then fully withdrawn prior to compacting the tampon blank (28) thus formed to its final tampon form.

11. A method according to Claim 10, wherein the strip-part (30) projecting out from said one side is inserted into said first mandrel (26;26') by means of said second mandrel (32).

12. A method according to Claim 10 or 11, wherein the strip-part (31) projecting out from said other side is inserted into said second mandrel (32) by means of a third mandrel.

13. A method according to Claim 10 or 11, wherein a withdrawal thread (33) is attached to the strip-part (31) which projects out from said other side.

14. A method according to Claim 13, wherein the strip-part (31) to which the withdrawal thread (33) is attached is drawn into the second mandrel (32) by means of said withdrawal thread.

15. A method according to any one of Claims 10-14, wherein said material string (20) is formed, at least partially, on the layer-material strip (23) by supplying discrete particles or fibres of absorbent material to a region on one side of said strip and maintaining a subpressure in the same region on the opposite side of the strip.

16. A method according to Claim 15, wherein at least an essential part of said absorbent material consists of defibrated paper pulp or fluff pulp.

17. A method according to Claim 16, wherein said pulp is unbleached.

18. A method according to any one of Claims 10-17, wherein the material string (20;20') is terminated at a distance from at least one end of the strip (23) of layer material.

## Patentansprüche

1. Tampon, bestehend aus einem Körper mit im wesentlichen kreisförmigem Querschnitt, der durch einen Strang (20, 20') aus absorbierendem Material geformt ist, welches eine Weite aufweist, die im wesentlichen mit der Länge des Tampons übereinstimmt, und der verschiedene Male um eine sich quer zu der longitudinalen Richtung des Stranges hin erstreckenden Achse gewunden ist und aus einem dünnen flüssigkeitspermeablen Schichtmaterial (23), welches den gewundenen Strang (20, 20') umgibt und eine Ausdehnung in Richtung der oben genannten Achse aufweist, die größer ist als die Länge des Tampons, wobei die Bereiche (30, 31) des Schichtmaterials, welche sich über beide Seiten des gewundenen Stranges hinaus erstrecken, eingeschlagen werden und dem innerhalb der innersten Windung bestehendem Raum angepaßt werden um so eine Rolle zu formen, welche dann vorherrschend in ihrer radialen Richtung komprimiert wird um den oben genannten Körper zu formen, wobei das Schichtmaterial (23) aus einem länglichen Band besteht, welches zusammen mit dem Strang (20, 20') derart zusammengewunden wird, so daß eine Rolle entsteht, deren Windungen aus einzelnen Schichten des besagten Bandes (23) bestehen, die eine aus dem Strang (20, 20') des Absorbtionsmaterials bestehende Füllung aufweisen, die zwischen den Schichten angeordnet ist.

2. Tampon nach Anspruch 1, **dadurch gekennzeichnet**, daß der Strang (20') bogenförmig gekrümmt ist und wobei der Tamponvorkörper in eine gestumpft konische rollenartige Form gewunden ist, in welcher die durch einzelne Schichten des Bandes (23) getrennten Strangwicklungen im wesentlichen zentrisch übereinander angeordnet sind.

3. Tampon nach Anspruch 2, **dadurch gekennzeichnet**, daß der Strang (20') auf dessen länger gebogenen Seite dünner ausgebildet ist.

4. Tampon nach einem der Ansprüche 2 bis 3, **dadurch gekennzeichnet**, daß der Strang (20') im wesentlichen benachbart zu dessen länger gebogenen Seite ein Material enthält, welches eine intrinsische Elastizität aufweist, die im wesentlichen unabhängig von Feuchtigkeitsbedingungen ist, so daß der Tampon das Bestreben hat sich auszudehnen und in seine ursprüngliche, vor der Kompression aufweisende Gestalt zurückzukehren, dies selbst in einem im wesentlichen trockenen Zustand.

5. Tampon nach einem der Ansprüche 1 bis 4, **dadurch gekennzeichnet**, daß der Strang (20, 20') zumindest teilweise aus diskreten Teilchen oder Fasern eines Absorbtionsmaterials besteht.

6. Tampon nach Anspruch 5, **dadurch gekennzeichnet**, daß zumindest ein wesentlicher Teil des Absorbtionsmaterials aus defibrierter Papiermasse oder aus Zellstoff besteht.

7. Tampon nach Anspruch 6, **dadurch gekennzeichnet**, daß die Papiermasse oder der Zellstoff ungebleicht sind.

8. Tampon nach einem der Ansprüche 1 bis 7, **dadurch gekennzeichnet**, daß der Strang (20, 20') mit Abstand von zumindest einem Ende des Bandes (23) des Schichtmaterials abschließt.

9. Tampon nach einem der Ansprüche 1 bis 8, **dadurch gekennzeichnet**, daß ein Endstück eines Zugbandes (33) sich durch ein Ende des Tampons (29) erstreckt und durch den inwendig verdrehten Teil (30) des Bandes an diesem Ende umgeben wird und an dem Teil (31) des Bandes befestigt wird, das auf der gegenüber liegenden Seite des Tampons inwendig verdreht ist und inwendig der innersten Windung des Tampons angeordnet ist.

10. Verfahren zur Herstellung eines Tampons nach Anspruch 1, bestehend aus der Formung eines Tamponvorkörpers (28) in Form eines Körpers, der einen im wesentlichen kreisförmigen Querschnitt aufweist und eine Füllung eines Absorbtionsmaterials enthält, die in einer Einfassung eines dünnen flüssigkeitspermeablen Schichtmaterials eingeschlossen ist, einer Verdichtung des Tamponvorkörpers bis in die endgültige Tamponform, wobei das die Füllung bildende Absorbtionsmaterial in der Form eines Stranges (20, 20'), welcher eine Weite aufweist, die zumindest im wesentlichen mit der Länge des fertiggestellten Tampons (29) übereinstimmt, auf ein Band (23) des Schichtmaterials aufgebracht wird, welches eine größte Weite aufweist, die über die Weite des Stranges (20, 20') hinausragt, wobei der Strang auf dem Band in räumlichem Abstand zu den longitudinalen Rändern des Bandes angeordnet ist, wobei das Band (23) des Schichtmaterials mit dem darauf befindlichen Strang (20, 20') des Absorbtionsmaterials zur Formung des Tamponvorkörpers (28) in mehreren Wicklungen auf einen ersten tubusförmigen Dorn (26, 26') aufgewickelt wird, wobei der Strang auf der dem Dorn zugewandten Seite des Bandes angeordnet ist; ein Teil (30) des auf einer Seite des Stranges (20, 20') hervorstehenden Bandes (23) mit dem benachbart zu der inneren Kante des genannten Teiles angeordneten Endes des Dornes in das Innere des ersten Dornes (26, 26') eingeführt wird; ein zweiter tubusförmiger Dorn (32) durch das oben genannte Dornende des ersten Dornes (26, 26') dergestalt eingeführt wird, daß er von dem Teil des Schichtmaterials (30), das in den ersten Dorn eingeführt wurde, umgeben wird; der erste Dorn wird daraufhin vollständig zurückgezogen; ein Teil (31) des über den Strang (20, 20') auf der anderen Seite überstehenden Bandes (23) wird in das Innere des zweiten Dornes eingeführt, wobei das Ende des zweiten Dornes (32) benachbart zu der inneren Kante des oben genannten Teil des Bandes angeordnet ist; und wobei der zweite Dorn (32) daraufhin vollständig zurückgezogen wird, bevor der Tamponvorkörper (28) verdichtet wird und in seine endgültige Tamponform gebracht wird.

11. Verfahren nach Anspruch 10, **dadurch gekennzeichnet**, daß der über eine Seite überstehende Teil (30) des Bandes mittels des zweiten Dornes (32) in den ersten Dorn (26, 26') eingeführt wird.

12. Verfahren nach einem der Ansprüche 10 bis 11, **dadurch gekennzeichnet**, daß der über die andere Seite hinausragende Teil (31) des Bandes mittels eines dritten Dornes in den zweiten Dorn (32) eingeführt wird.

13. Verfahren nach einem der Ansprüche 10 bis 11, **dadurch gekennzeichnet**, daß ein Zugband (33) an dem Teil (31) des Streifens, welcher über die andere Seite hervorsteht, angebracht ist.

14. Verfahren nach Anspruch 13, **dadurch gekennzeichnet**, daß der Teil (31) des Bandes, an welchem das Zugband (33) angebracht ist, mittels des Zugbandes (33) in den zweiten Dorn (32) eingeführt wird.

15. Verfahren nach einem der Ansprüche 10 bis 14, **dadurch gekennzeichnet**, daß der Materialstrang (20) zumindest teilweise auf dem Band (23) des Schichtmaterials ausgebildet wird, in dem diskrete Teilchen oder Fasern des Absorbtionsmaterials einem Bereich einer der Seiten des Bandes zugeführt werden und ein Unterdruck in dem gleichen Bereich auf der gegenüberliegenden Seite des Bandes aufrechterhalten wird.

16. Verfahren nach Anspruch 15, **dadurch gekennzeichnet**, daß zumindest ein wesentlicher Teil des Absorbtionsmaterials aus defibriertem Papiermaterial oder Zellstoff besteht.

17. Verfahren nach Anspruch 16, **dadurch gekennzeichnet**, daß das Papiermaterial oder der Zellstoff ungebleicht sind.

18. Verfahren nach einem der Ansprüche 10 bis 17, **dadurch gekennzeichnet**, daß der Materialstrang (20, 20') mit einem Abstand von mindestens einem der Enden des Streifens (23) des Schichtmaterials abschließt.

## Revendications

1. Un tampon comprenant un corps de section transversal sensiblement circulaire, formé par une mèche (20 ; 20') de matière absorbante présentant une largeur qui correspond sensiblement à la longueur du tampon et enroulée sur plusieurs tours autour d'un axe s'étendant transversalement à la direction longitudinale de la mèche, et par une matière (23) formant une couche mince perméable au liquide entourant la mèche enroulée (20 ; 20') et présentant une extension dans la direction dudit axe supérieur à la longueur du tampon, dans lequel des parties (30, 31) de la matière formant une couche, qui s'étendent à l'extérieur des deux côtés de la mèche enroulée sont tordues vers l'intérieur et placées dans un espace situé vers l'intérieur du tour le plus interne pour former un rouleau qui est ensuite comprimé de manière prédominante dans sa direction radiale afin de former ledit corps, caractérisé en ce que ladite matière formant une couche (23) comprend une bande allongée qui a été enroulée en même temps que ladite mèche (20 ; 20') de manière à former un rouleau, dont les tours comprennent des couches uniques de ladite bande (23) avec un remplissage formé à partir de la mèche (20 ; 20') de matière absorbante placée entre lesdites couches.

2. Un tampon selon la revendication 1, dans lequel la mèche (20') est cintrée en arc de cercle et dans lequel l'ébauche du tampon est enroulée sous la forme d'un rouleau tronconique dans lequel les tours de la mèche séparés par une couche unique de bande (23) sont placés au moins sensiblement de manière centrale l'un au-dessus de l'autre.

3. Un tampon selon la revendication 2, dans lequel la mèche (20') est amincie au niveau de son côté cintré le plus long.

4. Un tampon selon la revendication 2 ou 3, dans lequel la mèche (20') contient, au moins à proximité de son côté cintré le plus long, une matière qui présente une élasticité intrinsèque qui est essentiellement indépendante des conditions d'humidité telles que le tampon aura tendance à se dilater et à revenir à la forme qu'il avait avant d'être comprimé, même dans un état sensiblement sec.

5. Un tampon selon l'une quelconque des revendications 1 à 4, dans lequel ladite mèche (20 ; 20') est composée au moins partiellement de particules ou de fibres discrètes de matière absorbante.

6. Un tampon selon la revendication 5, dans lequel au moins une partie principale de ladite matière absorbante comprend de la pulpe de papier défibrée ou de la pulpe de peluche.

7. Un tampon selon la revendication 6, dans lequel ladite pulpe n'est pas blanchie.

8. Un tampon selon une quelconque des revendications 1 à 7, dans lequel ladite mèche (20 ; 20') se termine à distance d'au moins une extrémité de la bande de matière en forme de couche (23).

9. Un tampon selon une quelconque des revendications 1 à 8, dans lequel une partie d'extrémité d'un cordon de retrait (33) s'étend d'une extrémité du tampon (29), entouré par la partie de bande enroulée vers l'intérieur (30), à ladite extrémité et est fixée à la partie de bande (31) enroulée vers l'intérieur à l'extrémité opposée du tampon et placée dans le tampon à l'intérieur du tour le plus interne.

10. Un procédé pour fabriquer un tampon selon la revendication 1, comprenant le fait de former une ébauche de tampon (28) sous la forme d'un corps présentant une section transversale sensiblement circulaire et contenant un remplissage de matière absorbante enfermée dans un boîtier de matière formant une couche mince perméable au liquide et compacter l'ébauche de tampon à sa forme de tampon finale, procédé dans lequel la matière absorbante formant ledit remplissage est appliquée sous la forme d'une mèche (20 ; 20') qui présente une largeur correspondant au moins sensiblement à la longueur du tampon terminé (29) sur une bande (23) de ladite matière en forme de couche qui présente une largeur supérieure dépassant la largeur de la mèche (20 ; 20'), ladite mèche étant placée sur la bande en relation d'écartement avec les bords longitudinaux de ladite bande, dans lequel :
la bande (23) de matière en forme de couche présentant, disposée dessus, la mèche (20 ; 20') de matière absorbante pour former ladite ébauche de tampon (28) et bobiner sur plusieurs tours sur un premier mandrin tubulaire (26, 26') avec ladite mèche placé sur le côté de la bande faisant face audit mandrin ; une partie (30) de la bande (23) faisant saillie d'un côté de la mèche (20 ; 20'), l'extrémité du mandrin placée à proximité du bord interne de ladite partie étant insérée à l'intérieur dudit premier mandrin (26, 26') ; un second mandrin tubulaire (32) est insérée à travers ladite extrémité du premier mandrin (26, 26') de manière à être entouré par la partie de matière en forme de couche (30) insérée dans le premier mandrin ;
le premier mandrin est ensuite complètement retiré ;
une partie (31) de la bande (23) faisant saillie de la mèche (20 ; 20') de l'autre côté, l'extrémité du second mandrin (32) étant placée à proximité du bord interne de la partie, est insérée à l'intérieur du second mandrin ; et
le second mandrin (32) est alors complètement retiré avant de compacter ladite ébauche de tampon (28) ainsi formée à sa forme de tampon finale.

11. Un procédé selon la revendication 10, dans lequel la partie de bande (30) faisant saillie dudit premier côté est insérée dans ledit premier mandrin (26, 26') au moyen dudit second mandrin (32).

12. Un procédé selon la revendication 10 ou 11, dans lequel la partie de bande (31) faisant saillie dudit autre côté est insérée dans ledit second mandrin (32) au moyen d'un troisième mandrin.

13. Un procédé selon la revendication 10 ou 11, dans lequel un cordon de retrait (33) est fixé à la partie de bande (31) qui fait saillie dudit autre côté.

14. Un procédé selon la revendication 13, dans lequel la partie de bande (31) à laquelle est fixé le cordon de retrait (33) est tirée dans le second mandrin (32) au moyen dudit cordon de retrait.

15. Un procédé selon une quelconque des revendications 1 à 14, dans lequel ladite mèche de matière (20) est formée, au moins partiellement, sur la bande de matière en forme de couche (23) par l'amenée de particules ou fibres discrètes de matière absorbante vers une région d'un côté de ladite bande et par le maintien, dans la même région, d'une dépression sur le côté opposé de la bande.

16. Un procédé selon la revendication 15, dans lequel au moins une partie essentielle ou de ladite matière absorbante est constituée de pulpe de papier défibrée ou de pulpe de peluche.

17. Un procédé selon la revendication 16, dans lequel ladite pulpe n'est pas blanchie.

18. Un procédé selon une quelconque des revendications 1 à 17, dans lequel la mèche de matière (20 ; 20') se termine à distance d'au moins une extrémité de la bande (23) en forme de couche.
